# EUROPEAN PATENT SPECIFICATION

(11) **EP 3 294 155 B1**
(45) Date of publication and mention of the grant of the patent: **09.11.2022**
(21) Application number: 16739285.1
(22) Date of filing: 13.05.2016
(51) Int. Cl.: A61B 17/16

(54) **SYSTEM FOR ORTHOPEDIC IMPLANTATION PREPARATION**
SYSTEM ZUR VORBEREITUNG EINES ORTHOPÄDISCHEN IMPLANTATS
SYSTÈME DE PRÉPARATION D'IMPLANTATION ORTHOPÉDIQUE

(30) Priority: 13.05.2015 US 201562161024 P
(43) Date of publication of application: 21.03.2018
(73) Proprietor: Smith & Nephew, Inc., Memphis, TN 38116 (US)
(72) Inventor: DEES, Roger, Ryan, Senatobia, MS 38668 (US); YEAGER, Jeffrey, N., Nesbit, MS 38651 (US)
(74) Representative: Appleyard Lees IP LLP
(86) International application number: PCT/US2016/032346
(87) International publication number: WO 2016/183437

(56) References cited:
- WO-A1-2005/094693
- WO-A2-2004/026169
- US-A- 4 791 919
- US-A1- 2002 099 447
- US-A1- 2013 172 892

## Description

### BACKGROUND

Embodiments of the present application generally relate to preparatory instrumentation for implantation of an orthopedic implant or component in a bone. More particularly, but not exclusively, embodiments of the present application relate to instrumentation for implantation of a metaphyseal and/or diaphyseal implant or augment relative to, and with selectively limited freedom about, one or more reference axis(es).

Proper alignment of a replacement joint device, including components of the replacement joint device, often can contribute to attaining optimal wear resistance performance of the implanted joint device. Yet, anatomical variations present challenges in properly aligning the implant joint device for each patient. For example, during implant construct of knee replacement joints, challenges can arise with fitting a patient's intramedullary geometry with an implant, such as, for example, an intramedullary stem, while also fitting both the external geometry with a condylar replacing implant and the metaphyseal and/or diaphyseal geometry with an associated implant or augment component. Moreover, the addition of a metaphyseal and/or diaphyseal implant or augment to an implant construct often impairs the ability to adjustably fit the implant to the patient and/or attain proper alignment of the various components of the implant. Such difficulties can at times be attributed to the anatomy of the patient, the geometrical constraints of the implant, and/or constraints associated with the preparatory instrumentation. For example, geometrical constraints of the metaphyseal and/or diaphyseal implant or augment can include the inability to accommodate the placement or position of both the intramedullary stem and the condylar implant, which can attribute to difficulties in forming a junction mechanism for those, and possibly other, components of the implant.

Challenges associated with attaining proper alignment during an implant construct that involves a metaphyseal and/or diaphyseal implant or augment may have, at times, been resolved by compromises in terms of the placement of at least some components of the implant device, such as, for example, the location of the condylar implant. Yet, such compromises can result in less than optimal bone coverage, which can potentially compromise loading of the construct to the cortical rim of the bone. Other compromises can include reducing the stem size in order to offset the stem position, with the area vacated by such offsetting being made up with cement. Yet, such compromises can adversely impact the life of the implant, and may be, at least in part, attributable to failures relating to subsidence, loosening, stress-shielding factors, and increased stresses on the implant device, among other failures that are associated with compromised articulation positioning.

The integrity of the implant construct can therefore be adversely impacted if the bone is not shaped, during implant surgery, to accommodate the positioning of augmenting implants at locations in which the implanted augments, such as, for example, stems, sleeves, and cones, among other augments, will not interfere with the articular component and/or other augmenting implants. Yet, the different anatomies of patients often present challenges in the ability to position augmenting implants at optimal locations. For example, in order to achieve optimal bone coverage, the articular component stem connection (post) axis can need to be at a location that is different than the stem axis. Further, the inability to attain such positioning can lead to compromises in the structure, life span, and/or performance of the implanted device, among other compromises. US4791919A relates to a reamer for cutting out a surface of revolution in the proximal end of the tibia.

### BRIEF SUMMARY

According to the invention there is provided a forming tool and guide as set out in the appended claims. An aspect of the present application is a forming tool having an outer wall that has a distal end and a proximal end, the distal end having a connection member that is adapted to couple the forming tool to a bone preparation device that is configured to facilitate displacement of bone material. The forming tool also includes a guide slot that extends through the outer wall. The guide slot is sized to axially receive insertion of a guide that is structured for placement in an intramedullary canal. Additionally, the guide slot has a guide slot axis and is shaped for linear displacement of the forming tool about the received guide in one or more directions that are perpendicular to a longitudinal axis of the forming tool.

Another aspect of the present application is a forming tool having an outer wall that includes a connection member that is adapted to couple the forming tool to a bone preparation device to facilitate displacement of bone material. The forming tool further includes a guide slot that extends through the outer wall, the guide slot being asymmetrical in cross sectional shape along a guide slot axis of the guide slot. Additionally, the guide slot is sized to axially receive insertion of a guide that is structured for placement in an intramedullary canal.

Another aspect of the present application is a forming tool having a guide slot that extends through an outer wall of the forming tool, the guide slot being asymmetrical in cross sectional shape along a guide slot axis of the guide slot. The guide slot is adapted to axially receive insertion of a guide that is structured for placement in an intramedullary canal. Additionally, the guide slot is sized to accommodate linear displacement of the forming tool about the received guide in one or more directions perpendicular to a longitudinal axis of the forming tool. The forming tool also includes a bone preparation device that is coupled to the outer wall by a connection member, the bone preparation device being structured to displace bone material.

### BRIEF DESCRIPTION OF THE DRAWINGS

The description herein makes reference to the accompanying figures wherein like reference numerals refer to like parts throughout the several views.
Figure 1 illustrates a front perspective view of a forming tool for preparing a bone for implantation of an augment or implant device according to an illustrated embodiment of the present application.
Figure 2 illustrates a front perspective view of an orientation referencing instrument or guide that extends along a guide axis and which is inserted into an intramedullary canal of a patient.
Figure 3 illustrates a front perspective view of at least a portion of the guide shown in Figure 2 being positioned within the forming tool depicted in Figure 1.
Figure 4A illustrates a side perspective view of the forming tool shown in Figure 1 without a bone preparation device and having an exemplary first shape for a guide slot that accommodates symmetrical positioning of at least the forming tool, outside the scope of the claims.
Figure 4B illustrates a section view of the forming tool shown in Figures 3 and 4A, which has limited positional freedom, positioned over a tibial bone and about a referencing guide or instrument.
Figure 4C illustrates a section view of a forming tool with asymmetrical positional freedom positioned over a tibial bone and about a referencing guide or instrument.
Figure 4D illustrates a section view of a forming tool outside the scope of the claims with symmetrical positional freedom positioned over a tibial bone and about a referencing guide or instrument.
Figure *SA* illustrates a side perspective view of the forming tool shown in Figure 1 without a bone preparation device and having an exemplary second shape for a guide slot that accommodates asymmetrical positioning of at least the forming tool.
Figure 5B illustrates a second end view of the forming tool shown in Figure 5A.
Figure 6A illustrates a side perspective view of the forming tool shown in Figure 1 without a bone preparation device and having an exemplary third shape for a guide slot that accommodates asymmetrical positioning of at least the forming tool.
Figure 6B illustrates a second end view of the forming tool shown inFigure 6A.
Figure 7A illustrates a side perspective view of the forming tool shown in Figure 1 without a bone preparation device and having an exemplary fourth shape for a guide slot that accommodates asymmetrical positioning of at least the forming tool.
Figure 7B illustrates a second end view of the forming tool shown inFigure 7A.
Figure 8 illustrates an exemplary bone preparation device according to an illustrated embodiment of the present application.
Figure 9A illustrates a perspective view of a first exemplary femoral implant that includes a femoral component, an augment, and a stem.
Figure 9B illustrates a cross sectional view taken along line 9B-9B in Figure 9A and depicts asymmetrical freedom of the augment relative to the stem.
Figure 9C illustrates a cross sectional view taken along line 9C-9C in Figure 9A and depicts asymmetrical freedom of the augment relative to the stem.

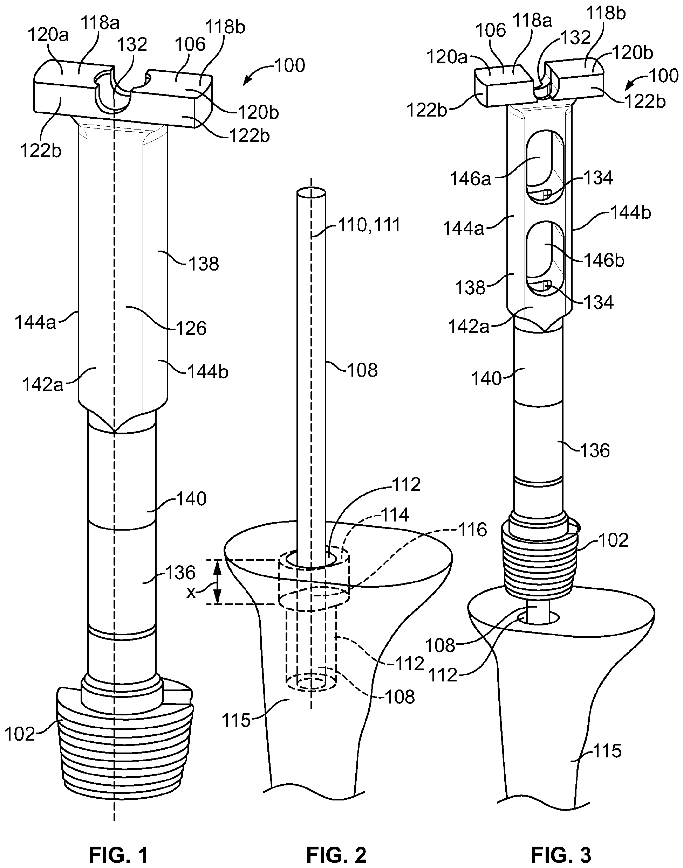

Figure 1OA illustrates a front perspective view of an exemplary tibial implant that includes a tibial tray, an augment, and a stem.
Figure 1OB illustrates a cross sectional view taken along line 1OB-1OB in Figure 1OA and depicts asymmetrical freedom of the augment relative to the stem and a tibial component.
Figure 1OC illustrates a bottom view of the exemplary tibial implant shown in Figure 1OA.

The foregoing summary, as well as the following detailed description will be better understood when read in conjunction with the appended drawings. It should be understood, however, that the present invention is not limited to the arrangements and instrumentalities shown in the attached drawings.

### DESCRIPTION OF THE ILLUSTRATED EMBODIMENTS

Certain terminology is used in the foregoing description for convenience and is not intended to be limiting. Words such as "upper," "lower," "top," "bottom," "first," and "second" designate directions in the drawings to which reference is made. This terminology includes the words specifically noted above, derivatives thereof, and words of similar import. Additionally, the words "a" and "one" are defined as including one or more of the referenced item unless specifically noted. The phrase "at least one of followed by a list of two or more items, such as "A, B or C," means any individual one of A, B or C, as well as any combination thereof.

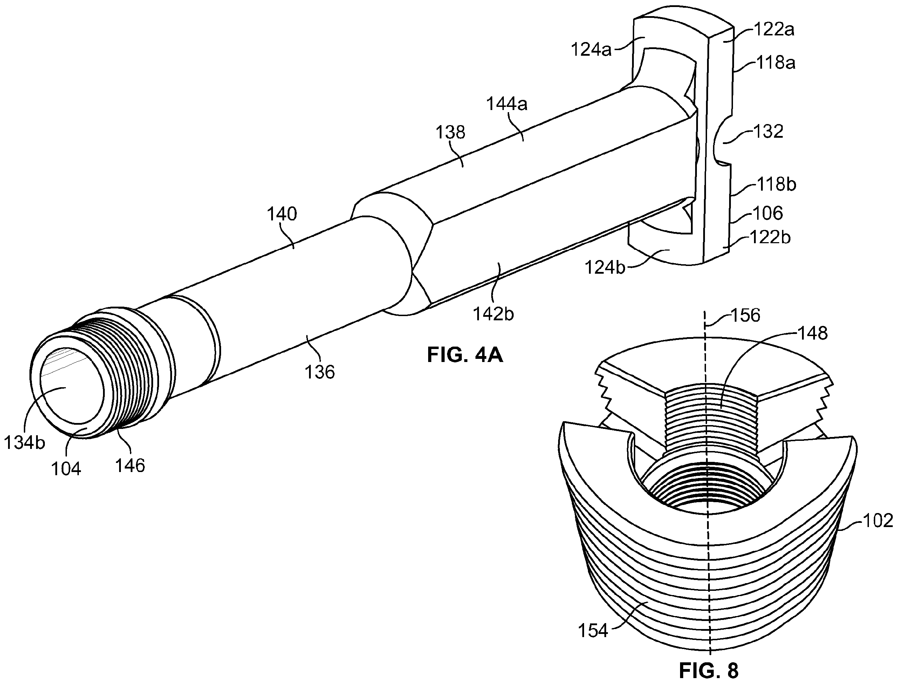

Figure 1 illustrates a front perspective view of a forming tool 100 for preparing a bone for implantation of an augment or implant device (collectively referred to as "augment") according to an illustrated embodiment of the present application. The forming tool 100 includes a bone preparation device 102 that is selectively coupled to a distal end 104 (Figure 4A) of the forming tool 100, as discussed below. The forming tool 100 also includes a proximal end 106 that is configured for engagement with an implantation tool, such as, for example, the proximal end 106 configured to be impacted or otherwise stricken by a hammer or mallet. Alternatively, the proximal end 106 is structured for coupling to another instrument that can assist in the formation of an augment opening in a bone, including, for example, an oscillating saw or drill.

The forming tool 100 is structured to receive insertion of, or otherwise engage, at least a portion of an orientation referencing instrument or guide 108, such as, for example, a intramedullary rod, trial stem, reamer, or offset rod, among other guides, as shown in Figures 2 and 3. According to the example provided in Figures 2 and 3, the guide 108 is a intramedullary rod that extends along a guide axis 110, which may, or may not, be generally aligned with a longitudinal axis 111 of the intramedullary canal 112 in the patient's bone 115. Further, according to the illustrated embodiment, the bone preparation device 102 is adapted to form an augment opening 114 having a depth (as indicated by "X" in Figure 2) in the bone 115. Further, the augment opening 114 is positioned about a central augment opening 116, as shown for example, in Figure 2, and can be generally at the same location, or can be both angularly and linearly offset from, a reference axis, such as, for example, the guide axis 110, the longitudinal axis 111 of the intramedullary canal 112, and/or one or more other axes of an implant device or components thereof.

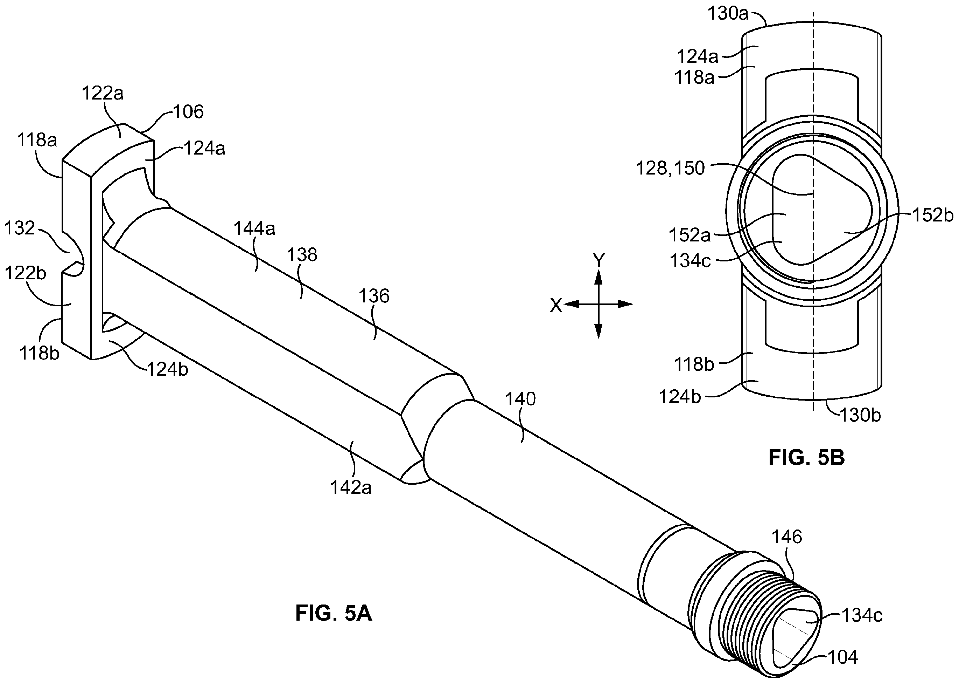

According to the illustrated embodiment, the proximal end 106 of the forming tool 100 includes one or more platforms 118a, 118b that are structured to be impacted during formation of the augment opening 114. Further, in the illustrated embodiment, a pair of platforms 118a, 118b extend outwardly away from a longitudinal axis 126 (Figure 1) of the forming tool 100 along a platform axis 128 (Figure 5B) that is generally perpendicular to the longitudinal axis 126 of the forming tool 100. According to such an embodiment, opposing ends 130a, 130b of the platforms 118a, 118b can be separated by a distance that provides the platforms 118a, 118b with an overall linear length that can enhance the size of the area that the implantation tool, such as, for example, a mallet, can impact or otherwise engage the forming tool 100. However, the platform(s) 118a, 118b can have a variety of other shapes and configurations, including, for example, having a round, non-round, circular, or triangular shape, among other shapes and/or segments of shapes.

According to the illustrated embodiment, the platforms 118a, 118b include an upper surface 120a, 120b that is configured to be directly or indirectly impacted or hit by the implantation tool. Outer edge walls 122a, 122b of the platforms 118a, 118b can extend from the upper surface 120a, 120b to a lower surface 124a, 124b of the platforms 118a, 118b. Further, the platforms 118a, 118b can have a thickness that at least assists in facilitating the ability of the platforms 118a, 118b to withstand the impact forces imparted upon at least the platforms 118a, 118b by the impact device. The platforms 118a, 118b can also include an aperture 132 that is in fluid communication with, and/or is an extension of, a guide slot 134 that extends through at least a portion of the forming tool 100, as shown in at least Figure 3, and as discussed below. In the illustrated embodiment, the aperture 132 can provide a user with visual access into the guide slot 134 so as to provide an indication of the position of at least the forming tool 100 relative to at least the guide 108 and/or the guide axis 110.

The forming tool 100 includes an outer wall 136 that extends between at least the platforms 118a, 118b and the distal end 104 of the forming tool 100. Further, the outer wall 136 may, or may not, have a unitary, monolithic structure. Further, according to certain embodiments, the size and shape of the outer wall 136 can vary along different portions of the forming tool 100. For example, according to the illustrated embodiment, a first section 138 of the forming tool 100 can generally extend from the lower surface 124a, 124b of the platforms 118a, 118b to a second section 140 of the forming tool 100. In the illustrated embodiment, the first section 138 of the forming tool 100 can be generally configured to enhance the ability of the user to grip or otherwise hold the forming tool 100. Thus, in the illustrated embodiment, the first section 138 can include opposing pairs of first walls 142a, 142b that are connected to each other by a pair of opposing second walls 144a, 144b. The first walls 142a, 142b and the second walls 144a, 144b can have a variety of shapes and sizes. For example, referencing Figure 1, according to certain embodiments, the opposing first walls 142a, 142b can both have a generally flat or straight shape that extends along a plane that is generally parallel to the longitudinal axis 126 of the forming tool 100. Further, according to certain embodiments, the second walls 144a, 144b can extend along a radius such that the second walls 144a, 144b have a curved or at least partially rounded shape. However, the first section 138 of the outer wall 136 can have a variety of other similar or dissimilar shapes, sizes, and configurations.

As shown in Figure 3, according to certain embodiments, the first section 138 of the outer wall 136 can include one or more orifices 146a, 146b that extend along portions of the first section 138 and through the first walls 142a, 142b. Additionally, according to the illustrated embodiments, the orifices 146a, 146b can each extend along parallel or non-parallel orifice axes that are perpendicular to the longitudinal axis 126 of the forming tool 100. Further, the orifices 146a, 146b can be positioned at a variety of other locations in addition to, or in lieu of, being positioned at the first section 138 of the outer wall 136, including, for example, extending through the second section 140. Additionally, the orifices 146a, 146b can have a variety of shapes and sizes. Further, such orifices 146a, 146b can facilitate the ability of the user to gauge the position of the forming tool 100 relative to a reference indicator or axis, such as, for example, but not limited to, the position of the forming tool 100 relative to the intramedullary canal 112, the guide 108, and/or guide axis 110.

According to certain embodiments, the second section 140 of the outer wall 136 can extend from the first section 138 in the general direction of the distal end 104 of the forming tool 100. The second section 140 may or may not have a size and shape similar to that of the first section 138. For example, according to the illustrated embodiment, the second section 140 can have a generally cylindrical shape of uniform or non-uniform shape or size. Alternatively, the second section 140, or a portion of the second section 140 can be eliminated, and instead the first section 138 of the outer wall 136 can generally extend to a connection member portion 146 of the forming tool 100, as shown in at least Figure 4A. Conversely, according to other embodiments, the first section 138, or a portion of the first section 138, of the outer wall 136 can be eliminated and/or replaced by the second section 140 of the outer wall 136.

The connection member 146 of the forming tool 100 can be configured to be coupled, directly or indirectly, to the bone preparation device 102. Further, the connection member 146 can be imparted with a variety of different configurations that can be utilized to securely couple the connection member 146, and thus the forming tool 100, to the bone preparation device 102. For example, as shown by at least Figures 4A and 8, the connection member 146 can include an external thread that is configured to mate an internal thread in an aperture 148 of the bone preparation device 102. According to other embodiments, the connection member 146 and bone preparation device 102 can be connected via insertion of a key or other projection from the forming tool 100 into a slot of, or otherwise pressed against, the bone preparation device 102, or vice versa. According to other embodiments, the connection member 146 and the bone preparation device 102 can be coupled together by a spring capture mechanism, or other biasing or retaining mechanism.

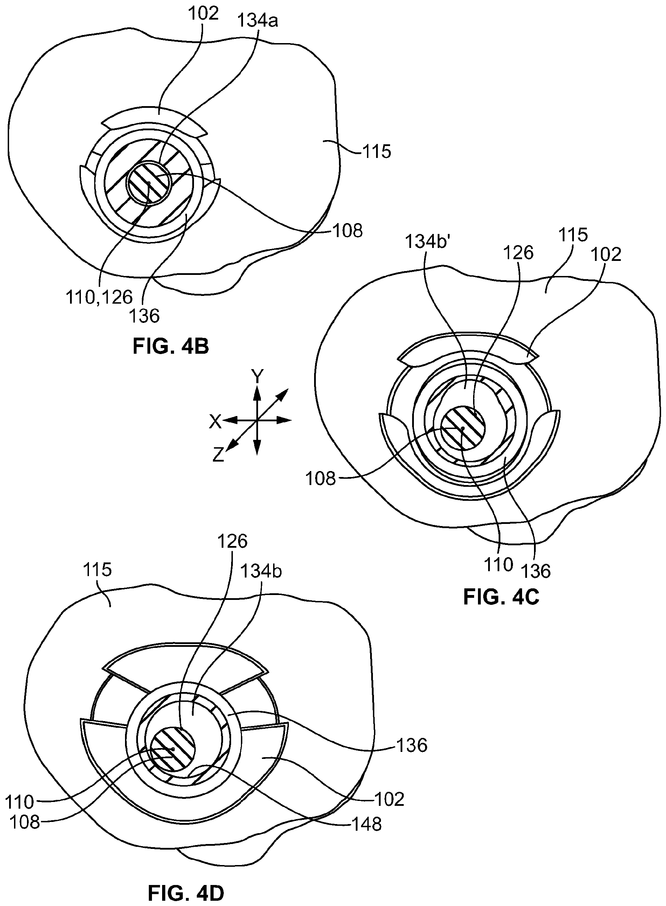

The forming tool 100 includes a guide slot 134 that extends through at least a portion of the forming tool 100, and which is sized to at least receive the insertion of at least a portion of the guide 108. As shown in Figure 4B, according to certain configurations, the guide slot 134a can have a cross sectional size and/or shape that is similar to that of the guide 108. Moreover, as shown in Figure 4B, according to certain embodiments, the guide slot 134a can have a size, such as an inner diameter, that is similar to the outer diameter of the guide 108 so that the guide slot 134a can receive insertion of the guide 108, but which allows for limited, if any, displacement of the forming tool 100 about the inserted guide 108. Thus, such similarities in the shape and/or sizes of the guide slot 134a and the guide 108 can limit or prevent adjustment in the location that the augment opening 114 is formed in the bone 115 via use of the forming tool 100.

Referencing Figures 4A, 4C, and 4D, according to other examples, wherein Figures 4A and 4D are outside the scope of the claims, the guide slot 134b, 134b' can have a size and/or shape that allows for adjustment in the position of the forming tool 100 relative to at least the location of the inserted guide 108, and therefore adjustment in the location at which the augment opening 114 can be formed in bone 115. For example, Figures 4A and 4D illustrate a forming tool 100 that includes a guide slot 134b having a shape similar to, but larger than, the guide slot 134a shown in Figure 4B. Such an increase in the size of the guide slot 134b can facilitate and/or increase the degree of the freedom to symmetrically displace the forming tool 100 about the inserted guide 108, and thus alter the position of at least the bone preparation device 102, and resulting augment opening 114, relative to at least the guide 108. Thus, while the smaller diameter guide slot 134a of Figure 4B can generally prevent altering the location of at least the forming tool 100 relative to the guide 108, the larger guide slot 134b shown in Figures 4A and 4D allows for the forming tool 100 to be moved about the guide 108 in a variety of different directions, thereby allowing for adjustment in at least the location at which the augment opening 114 can be formed in the bone 115 through use of the forming tool 100.

Figures 4C and 4D illustrate asymmetrical and symmetrical guide slots 134b, 134b' that are sized to permit the forming tool 100 to be displaced so that the guide 108 can be, for example, generally located in lower left portion of the guide slot 134b, 134b'. According to such an example, unlike the guide slot 134a shown in Figure 4B, the shape and/or size of the guide slots 134b, 134b' shown in Figures 4C and 4D permit displacement of the forming tool 100 such that the longitudinal axis 126 of the forming tool 100 can be offset from the guide axis 110 of the guide 108 along at least two axes (as indicated by the "X" and "Y" directions in Figure 4C), as well as in directions that are a combination of those axes (X, Y), among other axes. Further, as demonstrated by Figure 4D, in this exemplary embodiment, such freedom can allow for the forming tool 100 to be positioned such that the guide 108 contacts an inner wall 148 of the bone preparation device 102, thereby increasing the distance between the guide 108 and an opposing side of the inner wall 148. Further, such positioning can permit the augment to be implanted at a position in which the augment touches another component of the implant device, such as, for example, touches a tibial stem and/or a tray stem that extends from a tibial tray.

In addition to displacement along at least two axes (X, Y), and a combination thereof, according to certain embodiments, the guide slot 134 can also allow for a degree of adjustment or displacement in the angular orientation of the forming tool 100 along a third axis (as indicated by the "Z" direction in Figure 4C). Such angular displacement can, in at least certain situations, allow at least the longitudinal axis 126 of forming tool 100, and/or the central augment axis 116 of the resulting formed augment opening 114, to be non-parallel to at least the guide axis 110 of the guide 108.

Figures 5A-7B illustrate forming tools 100 having different shaped and sized guide slots 134c-e that can accommodate and/or limit displacement of the forming tool 100 about the guide 108. For example, Figures 5A-5B illustrate a guide slot 134c having a generally triangular or three sided shape. In the orientation shown in Figure 5B, the guide slot 134c is asymmetrical relative to a transversal midline 150 that is perpendicular to the longitudinal axis 126 of the illustrated forming tool 100, and which is generally parallel to the platform axis 128. However, the transversal midline 150 can be positioned at a variety of locations and/or orientations, including, for example, being offset from the longitudinal axis 126 and/or the platform axis 128. Further, according to certain embodiments, the transversal midline 150 may not be perpendicular to the longitudinal axis 126 of the illustrated forming tool 100 and/or can be non-parallel to the platform axis 128.

In the configuration depicted in Figures 5A and 5B, a first side 152a of the guide slot 134c relative to the transversal midline 150 can be configured to accommodate a larger degree of linear displacement of the forming tool 100 about the guide 108 along at least a first axis (as indicated by "X" in Figure 5B) than can be accommodated by an opposing, second side 152b of the guide slot 134c along that same axis. Conversely, the opposing second side 152b can be sized to accommodate a larger degree of linear displacement of the forming tool 100 about the guide 108 along at least a different, second axis (as indicated by "Y" in Figure 5B) than can be accommodated by the first side 152a of the guide slot 134c. Accordingly, the triangular shape of the guide slot 134c can also allow the first and second sides 152a, 152b of the guide slot 134c to accommodate different degrees of linear displacement of the forming tool 100 about the guide slot 134c along a combination of the first and second axes (X, Y). Similarly, the different shapes and/or sizes of the guide slot 134c can also allow the first and second sides 152a, 152b of the guide slot 134c to accommodate different degrees of rotational displacement of the forming tool 100 about the guide 108.

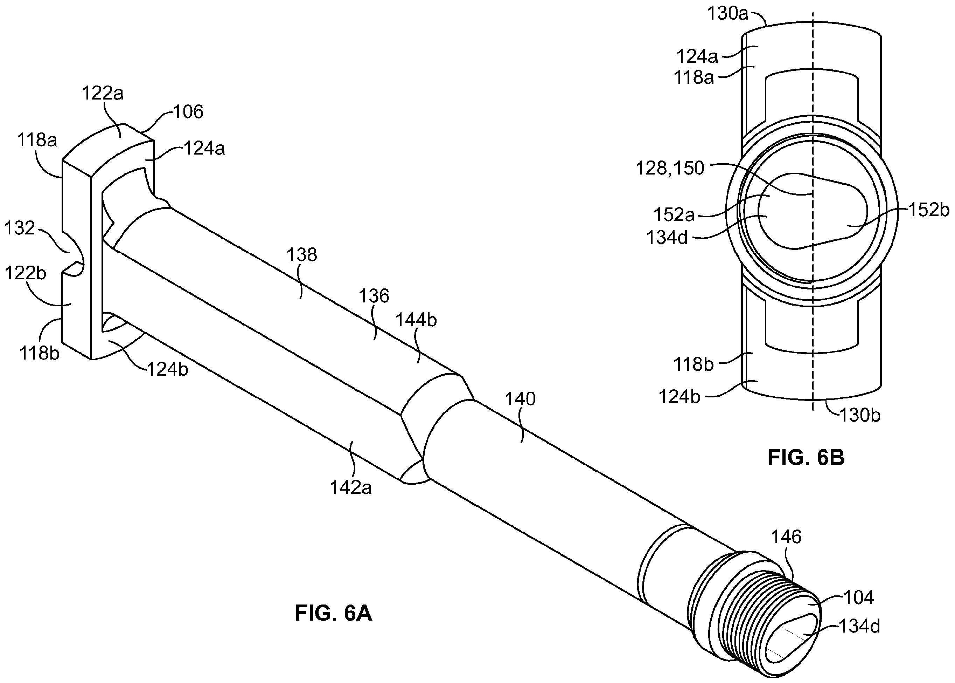

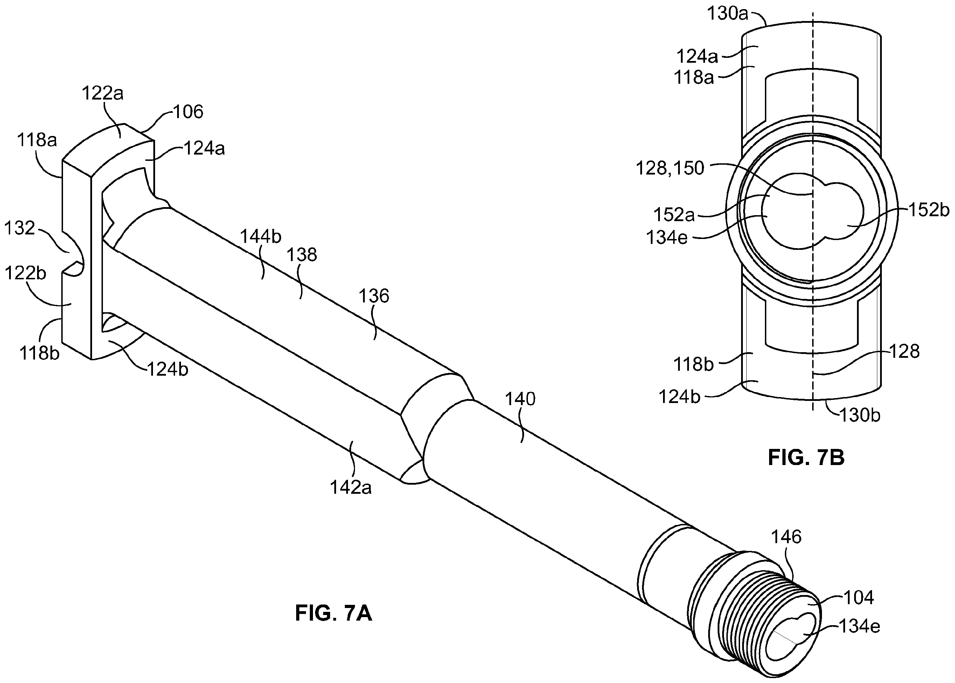

Similar to Figures 5A and 5B, the tapered shaped guide slot 134d shown in Figures 6A and 6B, and the different sized, intersecting circles of the guide slot 134e of Figures 7A and 7B, can also provide first and second sides 152a, 152b relative to the transversal midline 150 that can accommodate different degrees of linear and/or rotational displacement of the forming tool 100 relative to at least the guide 108.

Alternatively, according to other embodiments, the guide 108 can be adapted to receive insertion of at least a portion of the forming tool 100. According to such an alternative embodiment, the guide 108 can include an opening that receives insertion of at least a portion of the forming 100, the opening being configured to constrain the degree of freedom, whether symmetrical or asymmetrical, of linear and/or rotational displacement of at least the forming tool 100 relative to the guide 108.

Figure 8 illustrates an exemplary bone preparation device 102 that is structured for operable attachment to the connection member 146 of the forming tool 100 according to an illustrated embodiment of the present application. In the depicted embodiment, the bone preparation device 102 is a broach. According to such embodiments, the proximal end 106 of the forming tool 100 can be engaged by a tool, such as, for example, stricken by a mallet, such that the bone preparation device 102 is forced into the bone 115. The forming tool 100 can continue to be engaged by the tool until the bone preparation device 102 attains a particular depth and/or a particular shape is attained in the bone 115. Further, upon attaining a particular depth and/or shape in the bone 115, the bone preparation device 102 can be removed from the connection member 146 and replaced with a different sized bone preparation device 102, such as, a larger broach, and the process can be repeated until a particular size and/or shape is attained in the bone 115. However, a variety of other types of bone preparation devices 102 other than a broach can be used with the forming tool 100, including, for example, a rasp, reamer, or other cutting instrument.

In the depicted embodiment, the bone preparation device 102 includes an outer surface 154 that is adapted to facilitate displacement of the bone preparation device 102 into, and/or removal of, the surrounding bone 115. Further, according to certain embodiments, the outer surface 154 of the bone preparation device 102 can symmetrically or asymmetrical extend about a central cutting axis 154 of the bone preparation device 102. According to the illustrated embodiment, during use, the central cutting axis 154 can be generally positioned along with the longitudinal axis 126 of the forming tool 100.

The aperture 148 of the bone preparation device 102 can be configured to facilitate a secure connection between the connection member 146 and the bone preparation device 102. According to the illustrated embodiment, the aperture 148 can include an internal thread that is adapted to mate an external thread of the connection member 146. However, the connection member 146 and the bone preparation device 102 can be directly or indirectly coupled together in a variety of other manners, including, for example, through a slotted, spring capture or biasing arrangement, among other retention arrangements. Additionally, while the above example of the forming tool 100 is discussed in terms of use with a bone preparation device 102, a variety of other components can be attached to the forming tool 100 in a similar manner, including, but not limited to, trial components for the implant device.

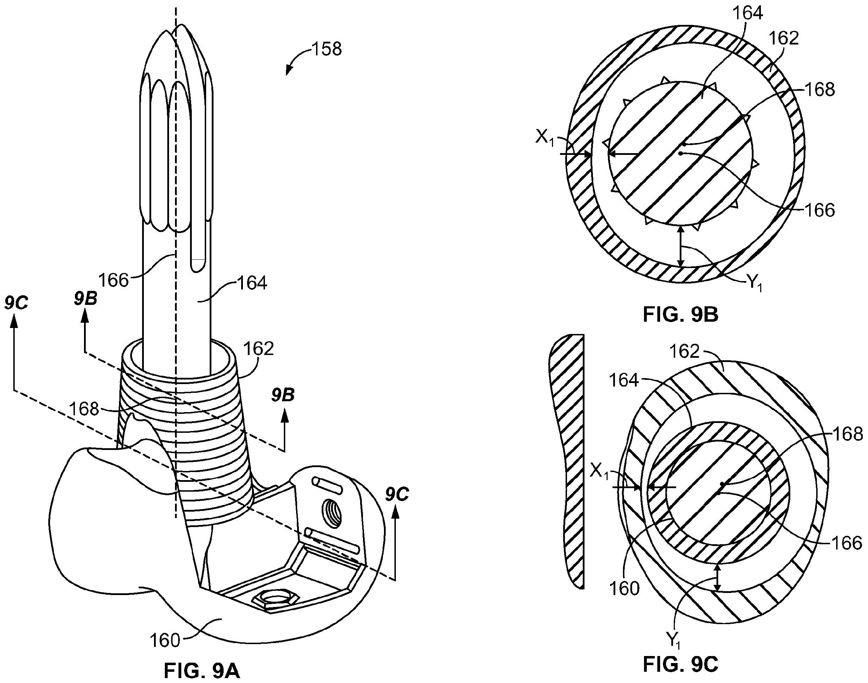

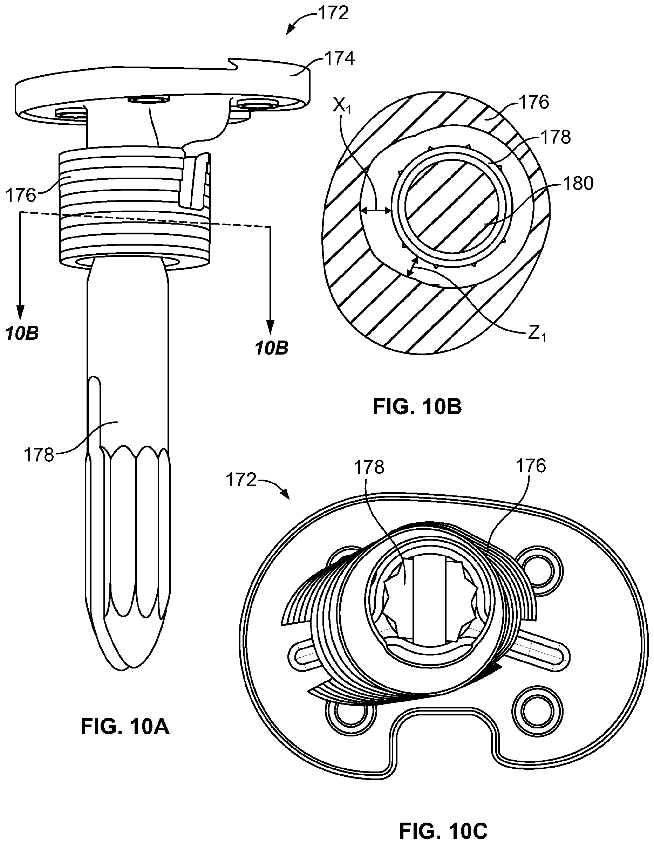

Figure 9A illustrates a perspective view of an exemplary femoral implant 158 that includes a femoral component 160, a femoral augment 162, and a stem 164. A variety of different augments can be used for the femoral augment 162, including, for example, a cone or sleeve augment, among other augments. As shown in Figure 9A, according to illustrated example, the stem 164 can extend along a stem axis 166. Further the femoral implant 158 can be configured or arranged such that the stem axis 166 may, or may not, be offset from a central augment axis 168 of the femoral augment 162.

Figure 9B, which is a cross sectional view taken along line 9B-9B in Figure 9A, provides an example of the asymmetrical positional freedom in the implant position(s) of the femoral augment 162 relative to the stem 164 that can be available via use of 9C-9C in Figure 9A, provides an example of the asymmetrical positional freedom in the implant position(s) of the femoral augment 162 relative to the stem 164 and a femoral component 160, such as, for example, a femoral stem of the femoral component 160, that can be available via use of the forming tool 100. For purposes of illustration, Figures 9B and 9C illustrate a first displacement direction (as indicated by "X₁" in Figures 9B and 9C) and a second displacement direction (as indicated by "Y₁" in Figures 9B and 9C) that can correspond to generally perpendicular directions in which the augment opening 114 can be positioned and/or formed in the bone 115, and thus the location at which the augment 162 can be displaced or offset relative to at least the stem 164, femoral component 160, and/or guide 108. In the depicted example, asymmetrical freedom in the positioning of the augment opening 114, and thus the location of the augment 162, can be, at least in part, provided by differences in the length along the first direction (X₁) and the length along the second direction (Y₁). Further, such asymmetrical freedom in the position of the augment 162 relative to at least the intramedullary canal 112, guide 108, stem 164, and/or femoral component 160 can be provided by use of a forming tool 100 having an asymmetrical guide slot 134, such as, for example, the asymmetrical guide slots 134c, 134d shown in at least Figures 5A-6B.

Further, although generally two displacement directions are discussed above, the asymmetrical positional freedom discussed above with respect to at least Figures 9A-9C can also extend to other directions, including directions that between or a combination of the first and second directions X₁, Y₁, and well as along a third axis, such as, for example, along the "Z" axis referenced in Figure 4C.

Figures 1OA and 1OC illustrate an exemplary tibial implant 172 that also includes a tibial tray 174, a tibial augment 176, and a stem 178. Additionally, Figure 1OB illustrates the augment 176 of the tibial implant 172 in which components of the tibial implant 172, including, for example, the tibia base or tray 174, tibial augment 176, stem 178 and/or other tibial components 180, as well as the integrity of those components and integrity criteria for at least the formed or shaped augmented opening 114 and/or associated bone 115 (Figure 2), result in an irregular, or inconsistent, degree of freedom being available for the placement of the forming tool 100 and/or the placement of the associated augment opening 114 or augment 176 in the bone 115. For example, as shown in Figure 1OB, the degree of freedom available for the formation of the augment opening 114, and the resulting relative positions of at least the augment 176 and the stem 178 and/or tray stem 180 along a first direction (as indicated by " X _{1"} in Figure 1OB) can be greater than the available degree of freedom along an second, adjacent and non-perpendicular direction (as indicated by "Z _{1"} in Figure 1OB).

While the invention has been described in connection with what is presently considered to be the most practical and preferred embodiment, it is to be understood that the invention is not to be limited to the disclosed embodiment(s), but on the contrary, is intended to cover various modifications included within the scope of the appended claims, which scope is to be accorded the broadest interpretation so as to encompass all such modifications as permitted under the law. Furthermore it should be understood that while the use of the word preferable, preferably, or preferred in the description above indicates that feature so described can be more desirable, it nonetheless may not be necessary and any embodiment lacking the same may be contemplated as within the scope of the invention, that scope being defined by the claims that follow. In reading the claims it is intended that when words such as "a," "an," "at least one" and "at least a portion" are used, there is no intention to limit the claim to only one item unless specifically stated to the contrary in the claim. Further, when the language "at least a portion" and/or "a portion" is used the item may include a portion and/or the entire item unless specifically stated to the contrary.

## Claims

1. A forming tool (100) and guide (108),
wherein the forming tool comprises:
an outer wall (136) having a distal end (104) and a proximal end (106), the distal end having a connection member (146) structured to couple the forming tool to a bone preparation device (102) to facilitate displacement of bone material; and
a guide slot (134) extending through the outer wall, the guide slot sized to axially receive insertion of a guide (108) that is structured for placement in an intramedullary canal (112), the guide slot having a guide slot axis, the guide slot shaped for linear displacement of the forming tool about the received guide in one or more directions perpendicular to a longitudinal axis (126) of the forming tool;
and wherein the guide is structured for placement in an intramedullary canal and structured to extend along the guide slot axis of the forming tool allowing for linear displacement of the forming tool about the received guide in one or more directions perpendicular to the longitudinal axis of the forming tool,
**characterised in that** the guide slot has an asymmetrical cross-sectional shape along the guide slot axis.

2. The forming tool and guide of claim 1, wherein a first side of a midline of the guide slot is adapted to accommodate a longer distance of linear displacement of the forming tool relative to the received guide along a first axis than can be accommodated by a second side of the midline of the guide slot along the first axis, and wherein the second side is adapted to accommodate a longer distance of linear displacement of the forming tool relative to the received guide along a second axis than can be accommodated by the first side along the second axis, and wherein the first axis is different than the second axis.

3. The forming tool and guide of any preceding claim, wherein the guide slot axis is linearly offset from the longitudinal axis of the forming tool.

4. The forming tool and guide of any preceding claim, wherein the proximal end of the outer wall includes one or more platforms (118a, 118b) structured to receive an impact force from an implantation tool.

5. The forming tool and guide of claim 4, wherein the one or more platforms comprises a pair of opposing arms (130a, 130b), the pair of opposing arms being generally perpendicular to the longitudinal axis (126) of the forming tool.

6. The forming tool and guide of claim 5, wherein the one or more platforms include an aperture (132) that is in fluid communication with the guide slot, the aperture structured to provide a visual indication of the location of at least the received guide relative to the guide slot.

7. The forming tool and guide of any preceding claim, wherein the connection member includes a threaded surface that mates a threaded surface of the bone preparation device.

8. The forming tool and guide of any one of claims 1 to 7, further comprising a bone preparation device coupled to the outer wall by a connection member, the bone preparation device being structured to displace bone material.

## Patentansprüche

1. Umformwerkzeug (100) und Führung (108),
wobei das Umformwerkzeug Folgendes umfasst:
eine Außenwand (136) mit einem distalen Ende (104) und einem proximalen Ende (106), wobei das distale Ende ein Verbindungsglied (146) hat, das dazu strukturiert ist, das Umformwerkzeug an eine Knochenvorbereitungsvorrichtung (102) zu koppeln, um die Verschiebung von Knochenmaterial zu ermöglichen, und
einen sich durch die Außenwand erstreckenden Führungsschlitz (134), wobei der Führungsschlitz zur axialen Aufnahme der Einführung einer Führung (108) bemessen ist, die zur Platzierung in einem intramedullären Kanal (112) strukturiert ist, wobei der Führungsschlitz eine Führungsschlitzachse hat, wobei der Führungsschlitz für die lineare Verschiebung des Umformwerkzeugs um die aufgenommene Führung in einer oder mehreren senkrecht zu einer Längsachse (126) des Umformwerkzeugs verlaufenden Richtungen gestaltet ist,
und wobei die Führung zur Platzierung in einem intramedullären Kanal strukturiert ist und dazu strukturiert ist, sich entlang der Führungsschlitzachse des Umformwerkzeugs zu erstrecken, wodurch die lineare Verschiebung des Umformwerkzeugs um die aufgenommene Führung in einer oder mehreren senkrecht zu der Längsachse des Umformwerkzeugs verlaufenden Richtungen gestattet ist,
**dadurch gekennzeichnet, dass** der Führungsschlitz entlang der Führungsschlitzachse eine asymmetrische Querschnittsform hat.

2. Umformwerkzeug und Führung nach Anspruch 1, wobei eine erste Seite einer Mittellinie des Führungsschlitzes dazu ausgeführt ist, eine längere lineare Verschiebungsstrecke des Umformwerkzeugs bezüglich der aufgenommenen Führung entlang einer ersten Achse aufzunehmen als von einer zweiten Seite der Mittellinie des Führungsschlitzes entlang der ersten Achse aufgenommen werden kann, und wobei die zweite Seite dazu ausgeführt ist, eine längere lineare Verschiebungsstrecke des Umformwerkzeugs bezüglich der aufgenommenen Führung entlang einer zweiten Achse aufzunehmen als von der ersten Seite entlang der zweiten Achse aufgenommen werden kann, und wobei sich die erste Achse von der zweiten Achse unterscheidet.

3. Umformwerkzeug und Führung nach einem der vorhergehenden Ansprüche, wobei die Führungsschlitzachse von der Längsachse des Umformwerkzeugs linear versetzt ist.

4. Umformwerkzeug und Führung nach einem der vorhergehenden Ansprüche, wobei das proximale Ende der Außenwand eine oder mehrere Plattformen (118a, 118b) aufweist, die zur Aufnahme einer Aufprallkraft eines Implantationswerkzeugs strukturiert sind.

5. Umformwerkzeug und Führung nach Anspruch 4, wobei die eine oder die mehreren Plattformen ein Paar gegenüberliegender Arme (130a, 130b) umfassen, wobei das Paar gegenüberliegender Arme allgemein senkrecht zu der Längsachse (126) des Umformwerkzeugs verlaufen.

6. Umformwerkzeug und Führung nach Anspruch 5, wobei die eine oder die mehreren Plattformen eine Öffnung (132) aufweisen, die in Fluidverbindung mit dem Führungsschlitz steht, wobei die Öffnung zur Bereitstellung einer visuellen Angabe des Ortes mindestens der aufgenommenen Führung bezüglich des Führungsschlitzes strukturiert ist.

7. Umformwerkzeug und Führung nach einem der vorhergehenden Ansprüche, wobei das Verbindungsglied eine Gewindefläche aufweist, die mit einer Gewindefläche der Knochenvorbereitungsvorrichtung koppelt.

8. Umformwerkzeug und Führung nach einem der Ansprüche 1 bis 7, ferner umfassend eine über ein Verbindungsglied an die Außenwand gekoppelte Knochenvorbereitungsvorrichtung, wobei die Knochenvorbereitungsvorrichtung zum Verschieben von Knochenmaterial strukturiert ist.

## Revendications

1. Outil de formation (100) et guide (108),
l'outil de formation comprenant :
une paroi extérieure (136) comportant une extrémité distale (104) et une extrémité proximale (106), l'extrémité distale comportant un élément de raccordement (146) structuré pour accoupler l'outil de formation à un dispositif de préparation de l'os (102) afin de faciliter le déplacement de la matière osseuse ; et
une fente de guidage (134) s'étendant à travers la paroi extérieure, la fente de guidage étant dimensionnée pour recevoir axialement l'insertion d'un guide (108) qui est structuré pour le placement dans un canal intramédullaire (112), la fente de guidage ayant un axe de fente de guidage, la fente de guidage étant façonnée pour un déplacement linéaire de l'outil de formation autour du guide reçu dans une ou plusieurs directions perpendiculaires à un axe longitudinal (126) de l'outil de formation ;
et le guide étant structuré pour le placement dans un canal intramédullaire et structuré pour s'étendre le long de l'axe de fente de guidage de l'outil de formation de façon à permettre un déplacement linéaire de l'outil de formation autour du guide reçu dans une ou plusieurs directions perpendiculaires à l'axe longitudinal de l'outil de formation,
**caractérisé en ce que** la fente de guidage présente une forme en section transversale asymétrique le long de l'axe de fente de guidage.

2. Outil de formation et guide selon la revendication 1, dans lequel un premier côté d'une ligne médiane de la fente de guidage est conçu pour permettre une plus grande distance de déplacement linéaire de l'outil de formation par rapport au guide reçu le long d'un premier axe que la distance permise par un second côté de la ligne médiane de la fente de guidage le long du premier axe, et dans lequel le second côté est conçu pour permettre une plus grande distance de déplacement linéaire de l'outil de formation par rapport au guide reçu le long d'un second axe que la distance permise par le premier côté le long du second axe, et dans lequel le premier axe est différent du second axe.

3. Outil de formation et guide selon l'une quelconque des revendications précédentes, dans lequel l'axe de fente de guidage est décalé linéairement vis-à-vis de l'axe longitudinal de l'outil de formation.

4. Outil de formation et guide selon l'une quelconque des revendications précédentes, dans lequel l'extrémité proximale de la paroi extérieure comprend une ou plusieurs plateformes (118a, 118b) structurées pour recevoir une force d'impact provenant d'un outil d'implantation.

5. Outil de formation et guide selon la revendication 4, dans lequel la ou les plateformes comprennent une paire de bras opposés (130a, 130b), la paire de bras opposés étant globalement perpendiculaire à l'axe longitudinal (126) de l'outil de formation.

6. Outil de formation et guide selon la revendication 5, dans lequel la ou les plateformes comprennent une ouverture (132) qui est en communication fluidique avec la fente de guidage, l'ouverture étant structurée pour fournir une indication visuelle de l'emplacement au moins du guide reçu par rapport à la fente de guidage.

7. Outil de formation et guide selon l'une quelconque des revendications précédentes, dans lequel l'élément de raccordement comprend une surface filetée qui correspond à une surface filetée du dispositif de préparation de l'os.

8. Outil de formation et guide selon l'une quelconque des revendications 1 à 7, comprenant en outre un dispositif de préparation de l'os accouplé à la paroi extérieure par un élément de raccordement, le dispositif de préparation de l'os étant structuré pour déplacer de la matière osseuse.
